Europäisches Patentamt

(19) European Patent Office

Office européen des brevets

(11) Publication number: **0 073 006**

**B1**

(12) **EUROPEAN PATENT SPECIFICATION**

(45) Date of publication of patent specification: **06.08.86**

(21) Application number: **82107498.6**

(22) Date of filing: **17.08.82**

(51) Int. Cl.⁴: **A 61 K 9/10, A 61 K 9/66**

(54) Medicinal composition and method of making same.

(30) Priority: **21.08.81 US 294907**

(43) Date of publication of application:
**02.03.83 Bulletin 83/09**

(45) Publication of the grant of the patent:
**06.08.86 Bulletin 86/32**

(84) Designated Contracting States:
**AT BE CH DE FR GB IT LI NL SE**

(56) References cited:
**DE-B-1 167 794**
**DE-C- 973 095**
**GB-A- 919 193**
**US-A-3 351 531**

(73) Proprietor: **Tucker, William Gough**
**4391 Maumee Drive**
**Okemos Michigan 48864 (US)**

(72) Inventor: **Tucker, William Gough**
**4391 Maumee Drive**
**Okemos Michigan 48864 (US)**

(74) Representative: **Kuhnen, Wacker & Partner**
**Schneggstrasse 3-5 Postfach 1729**
**D-8050 Freising (DE)**

Courier Press, Leamington Spa, England.

**Description**

Background of the invention

As well pointed out in the literature, e.g. Drug Absorption, Chapter 53, pages 130—137, *Gastroenterology Volume 2*, Third Edition, W. B. Saunders Company, Philadelphia, Penn., 1976; and in the "Lancet", November 10, 1979, pages 1003, 1004, drug absorption by the human body is a very complex affair which is subject to many upsetting factors. Some orally ingested drugs cause irritation of the gastric and intestinal mucosa, or are destroyed by acid or enzymes in the digestive tract, or react with other drugs or food materials. Some quite useful drugs are limited by their toxic effect on the body when utilized in therapeutic dosages, e.g. vitamin A in its various forms causes hepatitis and other liver ailments when administered in large amounts by conventional delivery means, including intraveneous feeding.

Yet, it has been found that retinoids, i.e. the varous forms of vitamin A, can be useful to arrest or reverse the progression of premalignant cells and thus to delay or prevent the development of invasive malignancy, Spron et al, Prevention of Chemical Carcinogenesis by Vitamin A and Its Synthetic Analogs, Federation of American Societies for Experimental Biology, *Federation Proceedings, 35: 1332—1338*, 1976; and Sporn et al, Chemoprevention of Cancer with Retinoids, *Federation Proceedings, 38: 2528—2534*, 1979. Other studies have reached similar conclusions. A recent incomplete and unreported research study indicates possible activity of vitamin A palmitate in the reactivation lymphocytes. In early cancer patients, responsive lymphocytes will migrate to attack the tumor cell; whereas in later states of cancer the lymphocyte is unable to migrate to attack the tumor cell. After treatment with vitamin A palmitate, $C_{36}H_{60}O_2$, there is an indication that the lymphocyte regains responsiveness and again attacks the tumor cell.

It is well known that the delivery of lipid-soluble drugs, such as vitamin A palmitate follows the normal course of fat digestion and absorption, involving emulsification, hydrolysis, absorption and chylomicron transport, requiring passage through the liver. Vitamin A palmitate is lipid-soluble and can be administered orally in an oil as a solvent. After passage through the stomach, the drug is assailed by the bile acids, converted by hydrolysis, and re-esterified during uptake and storage of vitamin A by the liver, see Smith and Goodman, *Nutrition Reviews' Present Knowledge in Nutrition*, pages 64—72, Fourth Edition, The Nutrition Foundation, Inc., N.Y., Washington, 1976.

For vitamin A palmitate when taken orally to enter the blood stream, the drug must be acted upon by the bile acids, hydrolyzed to a different form, reduced to a critical size (less than 0,1 µm (1000 Å)) passed through the brush layer of the small intestines and passed through the liver. If the drug is not acted upon swiftly by the bile acids or if it is not reduced to the critical size, then it passes on through the intestine. For these and other reasons (such as delayed re-esterification in the liver), the effective dosage of the drug actually delivered to the thoracic duct upon normal oral ingestion varies from patient to patient and from time-to-time for each patient. If it were possible to deliver lipid-soluble drug directly to the blood stream, then therapeutic dosages of such drugs as vitamin A palmitate would become possible without the dangers of the normal delivery system, such as liver damage, and such delivery will be more efficient, more predictable and relatively free of side effects. If such a system, in addition, were capable of delivering water soluble drugs, such as ferrous sulfate or potassium chloride, then the same improved therapeutic effects could be obtained while minimizing the side effects of such drugs.

US—A—3,351,531 to Noznick et al discloses a process in which glutin is dispersed in a volatile acid or base, and the dispersed glutin is then dispersed in an oil or fat to form an oil and water dispersion, with the oil or fat being the carrier for the food or medicine or other material which is desired to be introduced by the encapsulated product. The oil and water dispersion is then dried by spray drying to drive off the acid or base with the oil being encapsulated in the glutin. The product is a timerelease material since the glutin film is insoluble in neutral aqueous solution, but is soluble in the stomach acids. The additives such as peppermint oil, are meant to stimulate the taste, and the product obviously activates the maximum enzymatic chemical degradation of the glutin possible from the time of entry into the mouth through the gastro-intestinal tract. This teaching cannot solve the problem to pass an unaltered pharmaceutical agent through the stomach without digestion and for passage into the body through the brush layer.

DE—A—1167794 discloses a mixture incorporating a lipid, Vitamin A and warm gelatin which is homogenized with a paste of starch and water. The product ends up as a suspension of lipid gelatin in a paste of the starch and water. This disclosure discloses a mixture which is readily digested in the stomach, but not transferred through the stomach without digestion.

Brief description of the present invention

The present invention now proposes a solution to the problem of delivery of retinoids (typically vitamin A palmitate) and other pharmaceuticals to the body. The delivery system of the present invention specifically involves the delivery of a drug dispersed in oil droplets of extremely minute size (predominantly less than 0,1 µm (1000 Å) in diameter) and in unchanged form to the brush layer of the small intestine for absorption directly into the thoracic duct and hence into the blood system. While the system of the present invention is well adapted to delivery of retinoids, as hereafter explained in detail, it can also serve to deliver other drugs, whether lipid-soluble or water-soluble.

The desired drug is dispersed in the oil, either by solution in the case of lipid-soluble drugs or by forming a water-in-oil emulsion in the case of water-soluble drugs, by an emulsion technique. The emulsion is formed of the oil containing the drug, water and a surfactant, preferably in a ratio of about one part surfactant, two parts water, and four parts oil, with the ingredients being mixed and homogenized to form an emulsion in which the minute oil droplets are each surrounded by an enveloping layer of water which is tenaciously held in position by the surface tension of the water itself.

The emulsion then preferably is mixed with and distributed throughout a mass of solid powder, preferably a low molecular weight polysaccharide, with each droplet or globule of oil retaining its own identity and size. The powder is then encapsulated for ingestion. Alternatively, the capsule can be lined with the polysaccharide, and the emulsion can then be poured or otherwise introduced into the lined capsule.

In either case, the polysaccharide serves a multiplicity of functions, i.e. to maintain the integrity of the surrounding capsule by absorbing the water of the initial emulsion, to prevent the invasion of ambient moisture during storage of the capsules, to promote shelf life of the emulsion.

Once ingested, the gelatin capsule and the polysaccharide are digested or broken down in the stomach and the released oil globules with the pharmaceutical therein pass unaltered through the stomach and into the small intestine. Apparently, the minute oil droplets are presented to the brush layer of the small intestine in unaltered form for passage directly therethrough into the thoracic duct. Since vitamin A palmitate, for example, when delivered as herein proposed, does not hydrolyze in the bile acids and does not require re-esterification in the liver, the drug in effect by-passes the liver. The advantages will be evident.

Detailed description of the preferred embodiment of the invention

The emulsion of this invention comprises oil droplets or globules of a size predominantly less than 0.1 μm (1000 Å) in diameter and preferably ranging from about 500 to about 1000 Angstroms (Å), i.e., from about 0.05 to about 0.1 μm, within which the administered drug is present. It has been determined experimentally that the techniques here proposed result in an emulsion which contains predominantly (on the order of 70%) globules of a size corresponding to 0,1 μm (1000 Å) or less.

While the exact delivery mechanism is not known at this time, it appears that globules of this size do not excite or trigger the enzyme bile action of the body. Thus, oil globules of the fine micellar size contemplated by this invention can be delivered without change from the stomach and can be directly absorbed at the brush layer of the small intestine for passage directly into the thoracic duct and therefore directly into the blood system for systemic circulation.

The importance of such direct absorption can be appreciated for example in vitamin A therapy. Retinoids, such as vitamin A palmitate, $C_{36}H_{60}O_2$, when dissolved in oil globules of such size, can—in effect—by-pass the liver, with the retinoid passing undisturbed and in pristine condition through the stomach, through the brush layer of the small intestine and directly into the thoracic duct. Since such saturated retinyl esters (previously formed for therapeutic purposes only upon reesterification in the liver) are transported into the body through the lymphatic route, the presentation of vitamin A palmitate to the thoracic duct through the technique of this invention yields the desired therapeutic agent at the desired location without passage through the liver. The same benefits of exact drug delivery in precise dosages at the most beneficial place can be obtained for other drugs in essentially the same fashion, whether such drugs are lipid-soluble or water-soluble.

The emulsion also comprises an enveloping layer of water surrounding each oil globule. This water layer can be observed microscopically, and it serves to prevent agglomeration of the oil particles into a single mass, once the emulsion has been formed. The water layer is formed and retained by the surface tension of the water component of the emulsion, and persists as the emulsion passes through the stomach and is presented to the brush layer of the intestine. While the absorption mechanism at the brush layer is not fully known at this time, there is reason to believe that the water layer is stripped away either immediately prior to or during passage of the globule through the intercellular space of the brush layer itself.

The pharmaceutical component of the emulsion is dissolved directly and in the desired dosage in the initial oil component in the case of lipid-soluble substances, such as vitamin A palmitate, procarbazine, and BCNU (bis-chloroethyl nitrosourea). Where the pharmaceutical component is water soluble, such as ferrous sulfate or potassium chloride, the desired dosage amount of the pharmaceutical is dissolved in water distributed internally of the oil globules during the formation of the emulsion by an emulsion inversion technique. The water soluble drug is first mixed with the oil phase, then the surfactant is added, followed by the water, so that the drug then preferentially dissolves into the water added by the surfactant. The result is a water-in-oil emulsion obtained by an emulsion inversion technique, with the water containing the drug, and sufficient water is present to retain the enveloping water layer on the oil globules. The exterior water may contain some of the drug, but it makes no difference in the final result, since the water layer persists, at least until the brush layer is reached.

The surfactant is presently believed necessary to the formation of the fine emulsions of this invention. Where the surfactant was not present, the oil globules predominantly were larger than the desired size, varied substantially in size, and tended to separate or layer upon standing. Even processing non-surfactant emulsions through a centrifuge resulted in globules ranging from about 0,1 µm (1,000 Å) to about 1 µm (10,000 Å).

The surfactant itself preferably is a natural gum, such as gum arabic or gum tragacanth. In the use of gum tragacanth, larger amounts of emulsion water are required, on the order of ten times the amount required for the use of gum arabic. Gum arabic normally includes an enzyme which preferably is destroyed by heating the gum to a temperature of about 80°C prior to its use in the present invention. It is possible to use synthetic surfactants, although they have some tendency to crystallize, which may cause some problems with the retention of the water layer enveloping the oil globules. For these reasons, gum arabic is the presently most preferred surfactant, and natural gum-type surfactants are preferred, although other surfactants including synthetic materials, such as "Tween" (a product and a registered trade mark of ICI United States) can be utilized, if desired.

The oil utilized is preferably a vegetable oil, such as corn oil, olive oil, peanut oil, castor oil or sesame oil, or a fish oil, such as fractionated salmon oil. Since the oil acts as the carrier for the pharmaceutical, it must pass through the stomach without dissolution or digestion, and any oil capable of forming the desired emulsion, and which has the desired degree of inertness and is not toxic to the body can be utilized.

The presently most preferred emulsion of the present invention—utilizing a surfactant, preferably gum arabic, sterile water and an oil, preferably sesame oil—contains these ingredients in a ratio of about 1:2:4 by volume, with the drug present at the chosen concentration. Where gum tragacanth is utilized as a surfactant, the preferred proportions are about 1:20:40.

The emulsion can be formed by a simple mortar and pestle manual manipulation, as shown by the following examples. Commercially, any desired emulsifying and homogenizing apparatus can be used. A homogenizer of the Gaulin type is preferred, and such a homogenizer having a very small high pressure orifice through which the emulsion is forced at pressures up to 690 bar (10,000 psi) can be utilized. Centrifugal emulsifiers or homogenizers can also be used, but it is difficult to produce the extremely finely divided emulsions of the present invention in commercial quantities in centrifugal apparatus.

Once the emulsion is formed, consisting of the oil globules in water with each globule bearing its adherent water film, the emulsion is admixed with a solid powder to form a coherent, moldable or formable mass in which the individual oil globules are distributed. Alternatively, the emulsion in bulk can be encapsulated within the powder by a technique hereafter described. The preferred powder is a low molecular weight polysaccharide, and the most preferred polysaccharide is dextran of an average 40,000 daltons, ranging from about 10,000 to about 80,000 daltons. Other polysaccharides can be utilized, such as commercially available cornstarch. Heta starch or hydroxyethyl starch can also be utilized as the solid powder.

The purpose of the powder is to provide an osmotic pressure substance which is capable of picking up the excess water from the initial emulsion, where the excess water is defined as that in excess of the total water present in the enveloping water layer surrounding each oil globule plus any water emulsified in the oil where a water-soluble drug is present. Further, the powder will absorb any water which might invade the capsule during storage of the emulsion prior to use and any water which might interfere with encapsulation of the emulsion in a water-soluble capsule.

The powder is added in an amount in excess of the amount of the emulsion, typically about four (4) times the volume of the emulsion. In the final emulsion-powder composite, the powder preferably will constitute from 75 to 95% of the total volume, and most preferably from 80% to 90%.

In one version of the invention, the emulsion, as finally formed, is sprayed over or otherwise introduced into admixture with the dextran or other powder which has been placed in a mixing bin. The bin contains a rotatable double ribbon agitator with millers to prevent lumping or particle re-aggregation. After the desired pharmaceutical concentration of emulsion is attained, the mixing is halted. The powder picks up the excess water from the emulsion, and the composite appears to be a solid, firm mass which is almost rock-like. When the mass is broken into a powder and is microscopically observed, it will be seen that the individual drug-containing oil globules are still present, each globule with its own water surface coating. The composite powder can be molded to a capsule-filling shape, or the loose powder can be used to fill a capsule in the conventional manner.

Alternatively, the powder (preferably dextran) can be sprayed into the open halves of a capsule to form a coating or layer on the interior thereof, and the liquid emulsion can be poured into one half of the lined capsule and covered with the other half.

In either version of the invention, the emulsion-powder composite may be encapsulated into a conventional gelatin capsule or other stomach-soluble enclosure. If the emulsion alone were enclosed in the capsule, the capsule would dissolve or disintegrate in the water present in the emulsion. The powder makes possible the encapsulation of the emulsion, protects the emulsion from any moisture entering the capsule during storage, and at the same time preserves the essential character of the emulsion, per se.

The encapsulation technique of the present invention enhances the stability of lipid-soluble drugs sufficiently to ensure Federal Drug Administration approval, protects the drug, whether lipid-soluble or water-soluble, from oxidation or light (where the capsule is opaque), insures exact dosage compliance, and assures delivery of the micellar globules and the drug present therein to the brush layer in unaltered form. After ingestion of the capsule, the gelatin outer layer dissolves in the stomach, and the powder is digested or broken down in the stomach. For example, dextran is broken down to fructose.

Example I
Preparation of emulsion

1 ml of gum arabic was placed in a mortar. 4 ml of sesame oil having 1 ml of Vitamin A palmitate (1,000,000 I.U.) dissolved therein was added to the mortar. The mixture was constantly stirred and worked with a pestle as 2 ml of sterile water was added to the mixture. After 30 minutes of stirring and working, the emulsion was observed under a microscope utilizing a micrometer scale. It was found that 70% of the globules were of a size less than 0,1 μm (1000 Å), 50% were of a size less than 0,08 μm (800 Å), and 1—2% were of a size less than 0,04 μm (400 Å). Each globule was encased in a layer of water adhered thereto by surface tension.

Example II
Preparation of powder

To the 8 ml of product obtained as in Example I above, 32 ml by volume of low molecular weight dextran (average molecular weight 40,000 daltons, ranging from 10,000 to 80,000 daltons) was added to the mortar and mixed and worked until a homogeneous mixture was obtained.

The final mixture had a gross appearance similar to a stone which would pulverize with pressure. Upon microscopic examination, the emulsion was still present as minute globules with the surface layer of water intact on each oil globule.

Example III

Examples I and II were repeated, with the substitution of BCNU (200 mg of dry powder) for the vitamin A palmitate. Identical results were obtained in the formation of the emulsion (Example I) and the solid mass (Example II).

Example IV

4 ml of sesame seed oil mixed with 300 mg of finely powdered ferrous sulfate was thoroughly mixed using a mortar and pestle. 1 cc of gum arabic was added as mixing continued, then 2 ml of water was added. After about 30 minutes of continuous mixing and working a finely divided emulsion was obtained. 28 ml of low molecular weight dextran was added and mixing was continued, all as in Example II. Upon microscopic examination, as in Example II, the same results were obtained, e.g. a dry powder in which emulsion globules of a size predominantly less than 0,1 μm (1000 Å) were present, each globule being surrounded by a surface water layer.

Example V

Example IV was repeated, but substituting 400 mg of KCl for the ferrous sulfate ingredient. The results obtained were identical with those of Example IV.

Example VI

20 male Swiss mice, each 6 months old, were divided into two groups, A and B, of 10 mice each. 0.1 mg of the emulsion of Example I was dropper-fed to each of the mice of Group A, 0.25 ml of Mugos product was fed by dropper to each of the mice of Group B. The dosage level fed each mouse was 125,000 international units of vitamin A palmitate.

60 minutes after feeding, each mouse was sacrificed by blood letting, utilizing the inner canthus technique. Each blood sample was labeled and centrifuged. The serum of each sample was tested by high performance liquid chromotography to determine the vitamin A palmitate content. The results were as follows:

| | Group A | | | Group B | |
|---|---|---|---|---|---|
| Mouse | Vitamin A palmitate content | | Mouse | Vitamin A palmitate content | |
| 1 | 23 | | 1 | 12 | |
| 2 | 30 | | 2 | 18 | |
| 3 | 21 | | 3 | 9 | |
| 4 | 26 | | 4 | 11 | |
| 5 | 31 | | 5 | 5 | |
| 6 | 26 | | 6 | 9 | |
| 7 | 21 | | 7 | 11 | |
| 8 | 18 | | 8 | 4 | |
| 9 | 30 | | 9 | 7 | |
| 10 | 29 | | 10 | 5 | |
| Average | 25.5 | | Average | 9.1 | |

Example VII

Twenty patients were selected for a study. On a first day, each patient was given Mugos vitamin A palmitate oil solution (containing sesame oil, gum arabic, and D-alpha-Tocopherol acetate and vitamin A palmitate). Each patient received a dosage of Mugos solution containing 10,000 International units of vitamin A palmitate at 09:00 hours. 3 ml of blood was withdrawn from each patient at 13:00 hours of the same day and again at 17:00 hours.

The same 20 patients, on another day, were given capsules filled with the composition of Example II, and each containing 10,000 International units of vitamin A palmitate. The capsules were taken at 09:00 hours, and 3 ml of blood was withdrawn from each patient at 13:00 hours of the same day and again at 17:00 hours.

The vitamin A palmitate content of each sample of each patient's blood was determined by liquid chromotography, and the following results were obtained.

**0 073 006**

Vitamin A palmitate content

| Patient No. | Mugos sample | | Example II sample | |
|---|---|---|---|---|
| | 13:00 hours | 17:00 hours | 13:00 hours | 17:00 hours |
| 1 | 3 | 1 | 5 | 2 |
| 2 | 1 | 0 | 7 | 2 |
| 3 | 1 | 0 | 5 | 3 |
| 4 | 2 | 0 | 5 | 2 |
| 5 | 1 | 0 | 3 | 0 |
| 6 | 3 | 0 | 8 | 3 |
| 7 | 1 | 0 | 7 | 3 |
| 8 | 1 | 0 | 7 | 3 |
| 9 | 2 | 1 | 8 | 2 |
| 10 | 1 | 0 | 5 | 1 |
| 11 | 1 | 0 | 5 | 2 |
| 12 | 1 | 0 | 7 | 2 |
| 13 | 2 | 0 | 7 | 2 |
| 14 | 1 | 0 | 8 | 3 |
| 15 | 1 | 0 | 6 | 2 |
| 16 | 1 | 1 | 7 | 2 |
| 17 | 2 | 0 | 7 | 3 |
| 18 | 2 | 0 | 8 | 3 |
| 19 | 1 | 0 | 8 | 3 |
| 20 | 1 | 0 | 8 | 2 |
| Average | 1.45 | 0.15 | 6.6 | 2.25 |

It will be seen that after 4 hours, the composition of the invention placed an average of 4.55 times as much vitamin A palmitate in the blood, and after 8 hours, an average of 15 times as much.

Example VIII

A primary emulsion was prepared as set forth in Example I above. The oil, pharmaceutical and surfactant ingredients were first emulsified with one-half of the water and then the remaining one-half of the water was added.

The emulsion contained:

| Ingredient | Amount |
|---|---|
| Vitamin A palmitate | 3 grams |
| Peanut Oil | 2 grams |
| Water | 4 grams |
| Gum arabic | 1 gram |

The final emulsion was added slowly with a syringe to 42 grams of starch in a blender during rotation. The powder was removed from the blender and packed into capsules. 102 capsules were made, each containing 500 mg of powder and each containing a unit dosage of 50,000 International units of vitamin A palmitate.

### Claims for the Contracting States: BE, CH, DE, FR, GB, IT, LI, NL, SE

1. An orally ingestible medicinal composition capable of delivering a pharmaceutical in its unaltered original state to the brush layer of the small intestine, comprising a plurality of individual oil globules each enveloped within a surrounding layer of water adhered to the globules by surface tension and serving to retain the globules in independent droplet form, said globules being predominantly of a size less than 0,1 µm (1000 Å) in diameter, a pharmaceutical component disposed within each globule, a water absorbent carrier powder preventing access of extraneous water to said globules while leaving intact the enveloping water layer, and a capsule within which said powder and said globules are retained for ingestion, said capsule and said powder being digestible or broken up in the stomach so that the oil globules and the pharmaceutical component therein pass unaltered into the small intestine.

2. A composition as claimed in claim 1, wherein the pharmaceutical component is lipid-soluble and is dissolved in the oil globules.

3. A composition as claimed in claim 1, wherein the pharmaceutical component is water soluble and is present in the oil globules as an emulsion of water in oil.

4. A composition as claimed in claim 1, wherein the powder component is a polysaccharide with an average molecular weight of 40,000 daltons.

5. A composition as claimed in claim 1, wherein the oil is a vegetable oil.

6. A composition as claimed in claim 1, wherein the oil is fractionated salmon oil.

7. A composition as claimed in claim 1, wherein the composition also contains a natural gum surfactant.

8. A composition as claimed in claim 1, wherein the capsule consists essentially of gelatin and contains from 75 to 95% polysaccharide and the balance being a unit dosage of a pharmaceutical present in an oil which is distributed in the polysaccharide as individual globules of a diameter predominantly less than 0,1 µm (1000 Å).

9. A composition as claimed in claim 1 or 8, wherein the capsule consists essentially of gelatin and contains from 75 to 95% dextran and the balance a unit dosage of vitamin A palmitate dissolved in an oil which is emulsified with gum arabic and water, the gum arabic:water:oil ratio being 1:2:4 by volume, and the oil being present as individual globules of a diameter predominantly less than 0,1 µm (1000 Å).

10. A method of making a medicinal composition as claimed in claim 1 comprising the steps of

a. forming an emulsion consisting essentially of an oil having a pharmaceutical dispersed therein, a surfactant and water,

b. homogenizing the emulsion to reduce the oil to individual pharmaceutical-containing globules having a diameter predominantly less than 0,1 µm (1000 Å)

c. mixing the homogenized emulsion with a quantity of a polysaccharide to provide a final mixture which is 80—90% polysaccharide and the balance emulsion and in which the pharmaceutical-containing globules are unaltered; and

d. encapsulating the mixture in individual dosage units to yield a desired dosage amount of the pharmaceutical.

11. A method as claimed in claim 10, wherein the pharmaceutical is lipid-soluble and is dissolved in the oil.

12. A method as claimed in claim 10, wherein the pharmaceutical is water-soluble and is present in the oil as a water-in-oil emulsion.

13. A method as claimed in claim 10, wherein the surfactant is a natural gum.

14. A method as claimed in claim 11, wherein the emulsion is formed by mixing the oil having the pharmaceutical dissolved therein with the surfactant, then adding the water with additional mixing.

15. A method as claimed in claim 12, wherein the emulsion is formed by first mixing the water-soluble pharmaceutical with the oil, then adding the water and surfactant with additional mixing.

16. A method as claimed in claim 10, wherein the surfactant:water:oil ratio is about 1:2:4 by volume.

17. A method as claimed in claim 16, wherein the surfactant is gum arabic, the oil is a vegetable oil, and the polysaccharide is dextran present in an amount equal to about four times by volume of the amount of emulsion.

18. A method of making an orally ingestible medicinal composition as claimed in claim 1 comprising the steps of

a. forming an emulsion consisting essentially of an oil having vitamin A palmitate dissolved therein, gum arabic and water, the ratio of gum arabic:water:oil being 1:2:4 by volume,

b. homogenizing the emulsion to reduce the oil to globules having a diameter ranging from 0,05 µm (500 Å) to 0,1 µm (1000 Å),

c. mixing the homogenized emulsion with a quantity of dextran powder with an average molecular weight of 40,000 daltons equivalent to about four times the volume of the emulsion, and

d. encapsulating in a gelatin capsule an amount of the emulsion-dextran mixture necessary to provide a desired unit dosage of vitamin A palmitate.

19. An orally ingestible medicinal composition comprising lipid solvent, water and lipid-soluble pharmaceutical ingredients characterized by the fact that the ingredients are incorporated into a physical form to be orally ingested and capable of delivering the pharmaceutical in its unaltered original state to the brush layer of the small intestine, the physical form of the ingredients being as follows:

(a) the lipid solvent is in the form of individual globules predominantly of a size less than 0,1 μm (1000 Å) in diameter;

(b) each of the globules contains the lipid-soluble pharmaceutical dissolved therein, and;

(c) each of the globules is enveloped within a surrounding layer of water adhered to the globule by surface tension, said water layer serving to maintain the globules in independent form and to present only a water surface to the stomach fluids.

**Claims for the Contracting State: AT**

1. A method of making an orally ingestible medicinal composition comprising the steps of

a. forming an emulsion consisting essentially of an oil having a pharmaceutical dispersed therein, a surfactant and water,

b. homogenizing the emulsion to reduce the oil to individual pharmaceutical-containing globules having a diameter predominantly less than 0,1 μm (1000 Å),

c. mixing the homogenized emulsion with a quantity of a polysaccharide to provide a final mixture which is 80—90% polysaccharide and the balance emulsion and in which the pharmaceutical-containing globules are unaltered; and

d. encapsulating the mixture in individual dosage units to yield a desired dosage amount of the pharmaceutical.

2. A method as claimed in claim 1, wherein the pharmaceutical is lipid-soluble and is dissolved in the oil.

3. A method as claimed in claim 1, wherein the pharmaceutical is water-soluble and is present in the oil as a water-in-oil emulsion.

4. A method as claimed in claim 1, wherein the surfactant is a natural gum.

5. A method as claimed in claim 2, wherein the emulsion is formed by mixing the oil having the pharmaceutical dissolved therein with the surfactant, then adding the water with additional mixing.

6. A method as claimed in claim 3, wherein the emulsion is formed by first mixing the water-soluble pharmaceutical with the oil, then adding the water and surfactant with additional mixing.

7. A method as claimed in claim 1, wherein the surfactant:water:oil ratio is about 1:2:4 by volume.

8. A method as claimed in claim 7, wherein the surfactant is gum arabic, the oil is a vegetable oil, and the polysaccharide is dextran present in an amount equal to about four times by volume of the amount of emulsion.

9. A method of making an orally ingestible medicinal composition as claimed in claim 1 comprising the steps of

a. forming an emulsion consisting essentially of an oil having vitamin A palmitate dissolved therein, gum arabic and water, the ratio of gum arabic:water:oil being 1:2:4 by volume,

b. homogenizing the emulsion to reduce the oil to globules having a diameter ranging from 0,05 μm (500 Å) to 0,1 μm (1000 Å),

c. mixing the homogenized emulsion with a quantity of dextran powder with an average molecular weight of 40,000 daltons equivalent to about four times the volume of the emulsion, and

d. encapsulating in a gelatin capsule an amount of the emulsion-dextran mixture necessary to provide a desired unit dosage of vitamin A palmitate.

10. A method of making an orally ingestible medicinal composition comprising the steps of

a. forming an emulsion consisting essentially of an oil having a pharmaceutical dispersed therein, a surfactant and water, and

b. homogenizing the emulsion to reduce the oil to individual pharmaceutical-containing globules having a diameter predominantly less than 0,1 μm (1000 Å), whereby each of the globules containing the lipid-soluble pharmaceutical dissolved therein is enveloped within a surrounding layer of water adhered to the globule by surface tension, said water layer serving to maintain the globules in independent form and to present only a water surface to the stomach fluids.

**Patentansprüche für die Vertragsstaaten: BE, CH, DE, FR, GB, IT, LI, NL, SE**

1. Ein oral einnehmbares medizinisches Präparat, das in der Lage ist, ein Arzneimittel in seinem unveränderten ursprünglichen Zustand der Bürsenschicht des Dünndarmes zuzuführen, enthaltend eine Vielzahl von einzelnen Ölkügelchen, die jeweils von einer umgegebenden Schicht aus Wasser umhüllt sind, die an den Kügelchen durch Oberflächenspannung haftet und dazu dient, die Kügelchen in unabhängiger Tropfenform beizubehalten, wobei die Kügelchen vorwiegend eine Größe von weniger als 0,1 μm (1.000 Å) im Durchschnitt ausweisen, eine Arzneimittelkomponente, die innerhalb jedes Kügelchens angeordnet ist, ein wasserabsorbierendes Trägerpulver, das den Zugang von äußerem Wasser zu den Kügelchen verhindert, während es die umhüllende Wasserschicht unberührt läßt, und eine Kapsel, innerhalb welcher das Pulver und die Kügelchen für die Verdauung beibehalten werden, wobei die Kapseln und das Pulver im Magen verdaubar oder abbaubar sind, so daß die Ölkügelchen und die Arzneimittelkomponente, die darin enthalten ist, unverändert in den Dünndarm gelangen.

2. Präparat nach Anspruch 1, dadurch gekennzeichnet, daß die Arzneimittelkomponente lipid-löslich ist und in den Ölkügelchen gelöst ist.

3. Präparat nach Anspruch 1, dadurch gekennzeichnet, daß die Arzneimittelkomponente wasserlöslich ist und in den Ölkügelchen als eine Emulsion von Wasser in Öl vorleigt.

4. Präparat nach Anspruch 1, dadurch gekennzeichnet, daß die Pulver-Komponente ein Polysaccharid mit einem durchschnittlichen Molekulargewicht von 40.000 Dalton ist.

5. Präparat nach Anspruch 1, dadurch gekennzeichnet, daß das Öl ein pflanzliches Öl ist.

6. Präparat nach Anspruch 1, dadurch gekennzeichnet, daß das Öl fraktioniertes Salmonöl ist.

7. Präparat nach Anspruch 1, dadurch gekennzeichnet, daß das Präparat ebenfalls einen Naturgummi als oberflächenaktives Mittel enthält.

8. Präparat nach Anspruch 1, dadurch gekennzeichnet, daß die Kapsel im wesentlichen aus Gelatine besteht und von 75 bis 95% Polysaccharid und als Rest eine Einheitsdosis einer Arzneimittelkomponente enthalt, die in einem Öl vorliegt, das in dem Polysaccharid als einzelne Kügelchen mit einem Durchmesser von überwiegend weniger als 0,1 µm (1.000 Å) verteilt ist.

9. Präparat nach Anspruch 1 oder 8, dadurch gekennzeichnet, daß die Kapsel im wesentlichen aus Gelatine besteht und von 75 bis 95% Dextran und als Rest eine Einheitsdosis von Vitamin A-palmitat enthält, das in einem Öl gelöst ist, welches mit Gummi-arabikum und Wasser emulgiert ist, wobei das Volumenverhältnis von Gummi-arabikum:Wasser:Öl 1:2:4 beträgt und das Öl als einzelne Kügelchen mit einem Durchmesser von überwiegend weniger als 0,1 µm (1.000 Å) vorliegt.

10. Verfahren zur Herstellung eines medizinischen Präparates gemäß Anspruch 1, umfassend die Stufen

a) Bildung einer Emulsion, die im wesentlichen aus einem Öl, das eine Arzneimittelkomponente darin dispergiert enthält, einem oberflächenaktiven Mittel und Wasser besteht,

b) Homogenisieren der Emulsion zur Reduzierung des Öls zu einzelnen, die Arzneimittelkomponente enthaltenden Kügelchen mit einem Durchmesser von überwiegend weniger als 0,1 µm (1.000 Å),

c) Mischen der homogenisierten Emulsion mit einer Menge eines Polysaccharides, um ein endgültiges Gemisch bereitzustellen, das 80 bis 90% Polysaccharid und als Rest Emulsion enthält und in welchem die die Arzneimittelkomponente enthaltenden Kügelchen unverändert sind, und

d) Verkapselung des Gemisches in einzelnen Dosierungseinheiten zur Erzielung einer gewünschten Dosierungsmenge der Arzneimittelkomponente.

11. Verfahren nach Anspruch 10, dadurch gekennzeichnet, daß die Arzneimittelkomponente lipid-löslich ist und in dem Öl gelöst ist.

12. Verfahren nach Anspruch 10, dadurch gekennzeichnet, daß die Arzneimittelkomponente wasserlöslich ist und in dem Öl als eine Wasser-in-Öl-Emulsion vorliegt.

13. Verfahren nach Anspruch 10, dadurch gekennzeichnet, daß das oberflächenaktive Mittel ein natürlicher Gummi ist.

14. Verfahren nach Anspruch 11, dadurch gekennzeichnet, daß die Emulsion durch Mischen des Öles, das die Arzneimittelkomponente darin gelöst enthält, mit dem oberflächenaktiven Mittel und anschließendes Zusetzen von Wasser unter zusätzlichem Mischen hergestellt wird.

15. Verfahren nach Anspruch 12, dadurch gekennzeichnet, daß die Emulsion durch erstes Mischen der wasserlöslichen Arzneimittelkomponente mit dem Öl, dann Zusetzen des Wassers und des oberflächenaktiven Mittels unter zusätzlichem Rühren gebildet wird.

16. Verfahren nach Anspruch 10, dadurch gekennzeichnet, daß das Volumenverhältnis von oberflächenaktivem Mittel:Wasser:Öl etwa 1:2:4 beträgt.

17. Verfahren nach Anspruch 16, dadurch gekennzeichnet, daß das oberflächenaktive Mittel Gummi-arabikum ist, das Öl ein pflanzliches Öl ist und das Polysaccharid Dextran ist, das in einer Volumenmenge vorliegt, die gleich bis etwa 4 mal so groß wie die Menge der Emulsion ist.

18. Verfahren zur Herstellung eines oral einnehmbaren medizinischen Präparates gemäß Anspruch 1, umfassend die Stufen

a) Bildung einer Emulsion, die im wesentlichen aus einem Öl, das Vitamin A-palmitat darin gelöst enthält, Gummi-arabikum und Wasser besteht, wobei das Volumenverhältnis von Gummi-arabikum:Wasser:Öl 1:2:4 beträgt,

b) Homogenisieren der Emulsion zur Reduzierung des Öls zu Kügelchen mit einem Durchmesser im Bereich von 0,05 µm (500 Å) bis 0,1 µm (1.000 Å),

c) Mischen der homogenisierten Emulsion mit einer Menge von Dextranpulver mit einem durchschnittlichen Molekulargewicht von 40.000 Dalton, die gleich bis etwa 4 mal so groß wie das Volumen der Emulsion ist, und

d) Verkapseln einer Menge des Emulsion-Dextran-Gemisches, die zur Bereitstellung einer gewünschten Einheitsdosis von Vitamin A-palmitat notwendig ist, in einer Gelatinekapsel.

19. Ein oral einnehmbares medizinisches Präparat, enthaltend Lipidlösungsmittel, Wasser und lipid-lösliche Arzneimittelbestandteile, dadurch gekennzeichnet, daß die Bestandteile in einer physikalischen Form verbunden sind, um oral eingenommen werden zu können und den Arzneimittelbestandteil in seinem unveränderten ursprünglichen Zustand der Bürstenschicht des Dünndarmes zuführen zu können, wobei die physikalische Form der Bestandteile folgende ist:

a) das Lipidlösungsmittel liegt in der Form von einzelnen Kügelchen von vorwiegend einer Größe von weniger als 0,1 µm (1.000 Å) im Durchmesser vor,

b) jedes der Kügelchen enthält darin gelöst den lipid-löslichen Arzneimittel-Bestandteil und

c) jedes der Kügelchen ist eingehüllt in eine umgebende Schicht aus Wasser, die an den Kügelchen durch Oberflächenspannung haftet, wobei die Wasserschicht dazu dient, die Kügelchen in unbeeinflußter Form aufrechtzuerhalten und den Magenflüssigkeiten nur eine Wasseroberfläche zu präsentieren.

**Patentansprüche für den Vertragsstaat: AT**

1. Verfahren zur Herstellung eines oral einnehmbaren medizinischen Präparates, umfassend die Stufen

a) Bildung einer Emulsion, die im wesentlichen aus einem Öl, das eine Arzneimittelkomponente darin dispergiert enthält, einem oberflächenaktiven Mittel und Wasser besteht,

b) Homogenisieren der Emulsion zur Reduzierung des Öls zu einzelnen, die Arzneimittelkomponente enthaltenden Kügelchen mit einem Durchmesser von überwiegend weniger als 0,1 µm (1.000 Å),

c) Mischen der homogenisierten Emulsion mit einer Menge eines Polysaccharides, um ein endgültiges Gemisch bereitzustellen, das 80 bis 90% Polysaccharid und als Rest Emulsion enthält und in welchem die die Arzneimittelkomponente enthaltenden Kügelchen unverändert sind, und

d) Verkapselung des Gemisches in einzelnen Dosierungseinheiten zur Erzielung einer gewünschten Dosierungsmenge der Arzneimittelkomponente.

2. Verfahren nach Anspruch 1, dadurch gekennzeichnet, daß die Arzneimittelkomponente lipid-löslich ist und in dem Öl gelöst ist.

3. Verfahren nach Anspruch 1, dadurch gekennzeichnet, daß die Arzneimittelkomponente wasserlöslich ist und in dem Öl als eine Wasser-in-Öl-Emulsion vorliegt.

4. Verfahren nach Anspruch 1, dadurch gekennzeichnet, daß das oberflächenaktive Mittel ein natürlicher Gummi ist.

5. Verfahren nach Anspruch 2, dadurch gekennzeichnet, daß die Emulsion durch Mischen des Öles, das die Arzneimittelkomponente darin gelöst enthält, mit dem oberflächenaktiven Mittel und anschließendes Zusetzen von Wasser unter zusätzliches Mischen hergestellt wird.

6. Verfahren nach Anspruch 3, dadurch gekennzeichnet, daß die Emulsion durch erstes Mischen der wasserlöslichen Arzneimittelkomponente mit dem Öl, dann Zusetzen des Wassers und des oberflächenaktiven Mittels unter zusätzlichem Rühren gebildet wird.

7. Verfahren nach Anspruch 1, dadurch gekennzeichnet, daß das Volumenverhältnis von oberflächenaktivem Mittel:Wasser:Öl etwa 1:2:4 beträgt.

8. Verfahren nach Anspruch 7, dadurch gekennzeichnet, daß das oberflächenaktive Mittel Gummi-arabikum ist, das Öl ein pflanzliches Öl ist und das Polysaccharid Dextran ist, das in einer Volumenmenge vorliegt, die gleich bis etwa 4 mal so groß wie die Menge der Emulsion ist.

9. Verfahren zur Herstellung eines oral einnehmbaren medizinischen Präparates gemäß Anspruch 1, umfassend die Stufen

a) Bildung einer Emulsion, die im wesentlichen aus einem Öl, das Vitamin A-palmitat darin gelöst enthält, Gummi-arabikum und Wasser besteht, wobei das Volumenverhältnis von Gummi-arabikum:Wasser:Öl 1:2:4 beträgt,

b) Homogenisieren der Emulsion zur Reduzierung des Öls zu Kügelchen mit einem Durchmesser im Bereich von 0,05 µm (500 Å) bis 0,1 µm (1.000 Å),

c) Mischen der homogenisierten Emulsion mit einer Menge von Dextranpulver mit einem durchschnittlichen Molekulargewicht von 40.000 Dalton, die gleich bis etwa 4 mal so groß wie das Volumen der Emulsion ist, und

d) Verkapseln einer Menge des Emulsion-Dextran-Gemisches, die zur Bereitstellung einer gewünschten Einheitsdosis von Vitamin A-palmitat notwendig ist, in einer Gelatinekapsel.

10. Verfahren zur Herstellung eines oral einnehmbaren medizinischen Präparates, umfassend die Stufen

a) Bildung einer Emulsion, die im wesentlichen aus einem Öl, das darin dispergiert eine Arzneimittelkomponente enthält, einem oberflächenaktiven Mittel und Wasser besteht, und

b) Homogenisieren der Emulsion, um das Öl zu einzelnen, die Arzneimittelkomponente enthaltenden Kügelchen mit einem Durchmesser von überwiegend weniger als 0,1 µm (1.000 Å) zu reduzieren, wobei jedes der Kügelchen, das die lipid-lösliche Arzneimittelkomponente gelöst darin enthält, in einer umgebenden Schicht von Wasser, die an den Kügelchen durch Oberflächenspannung haftet, eingehüllt ist, wobei die Wasserschicht dazu dient, die Kügelchen in unbeeinflußter Form aufrechtzuerhalten und den Magenflüssigkeiten nur eine Wasseroberfläche zu präsentieren.

**Revendications pour les Etats Contractants: BE, CH, DE, FR, GB, IT, LI, NL, SE**

1. Composition médicinale pouvant être ingérée par voie orale, capable de délivrer une substance pharmaceutique dans son état initial non modifié à la couche en brosse de l'intestin grêle, comprenant plusieurs globules individuels d'huile enveloppés chacun d'une couche périphérique d'eau adhérant aux globules par la tension superficielle et servant à retenir les globules sous la forme indépendante de gouttelettes, lesdits globules étant principalement d'un diamètre inférieur à 0,1 µm (1000 Å), un composant pharmaceutique disposé dans chaque globule, une poudre de support absorbant l'eau interdisant à l'eau externe l'accès auxdits globules tout en laissant intacte la couche d'eau enveloppante, et une capsule dans laquelle ladite poudre et lesdits globules sont retenus en vue de l'ingestion, ladite capsule et ladite poudre pouvant être digérées ou dialoquées dans l'estomac de manière que les globules d'huile et le composant pharmaceutique qui s'y trouve passent dans l'intestin grêle sans être modifiés.

2. Composition suivant la revendication 1, dans laquelle le composant pharmaceutique est soluble dans les lipides et est dissous dans les globules d'huile.

3. Composition suivant la revendication 1, dans laquelle le composant pharmaceutique est soluble dans l'eau et est présent dans les globules d'huile sous la forme d'une émulsion eau-dans-huile.

4. Composition suivant la revendication 1, dans laquelle le composant de la poudre est un polysaccharide ayant un poids moléculaire moyen de 40 000 daltons.

5. Composition suivant la revendication 1, dans laquelle l'huile est une huile végétale.

6. Composition suivant la revendication 1, dans laquelle l'huile est de l'huile de saumon fractionnée.

7. Composition suivant la revendication 1, qui contient aussi comme surfactant une gomme naturelle.

8. Composition suivant la revendication 1, dans laquelle la capsule est essentiellement formée de gélatine et contient 75 à 95% de polysaccharide, le reste étant une dose unitaire d'un composé pharmaceutique présent dans une huile qui est distribuée dans le polysaccharide sous la forme de globules individuels ayant un diamètre principalement inférieur à 0,1 µm (1000·Å).

9. Composition suivant la revendication 1 ou 8, dans laquelle la capsule est essentiellement formée de gélatine et contient 75 à 95% de dextrane, le reste étant une dose unitaire de palmitate de vitamine A dissous dans une huile qui est émulsionnée avec de la gomme arabique et de l'eau, la proportion gomme arabique:eau:huile étant de 1:2:4 en volume et l'huile étant présente sous la forme de globules individuels ayant un diamètre principalement inférieur à 0,1 µm (1000 Å).

10. Procédé de préparation d'une composition médicinale suivant la revendication 1, comprenant les étapes qui consistent

a. à former une émulsion consistant essentiellement en une huile dans laquelle est dispersée une substance pharmaceutique, un surfactant et de l'eau,

b. à homogénéiser l'émulsion pour réduire l'huile en globules individuels contenant la substance pharmaceutique, ayant un diamètre principalement inférieur à 0,1 µm (1000 Å),

c. à mélanger l'émulsion homogénéisée avec une quantité d'un polysaccharide donnant un mélange final qui contient 80 à 90% de polysaccharide, le reste étant l'émulsion, et dans lequel les globules contenant la substance pharmaceutique sont intacts; et

d. à encapsuler le mélange en unités dosées individuelles pour obtenir une quantité dosée désirée de la substance pharmaceutique.

11. Procédé suivant la revendication 10, dans lequel la substance pharmaceutique est soluble dans les lipides et est dissoute dans l'huile.

12. Procédé suivant la revendication 10, dans lequel la substance pharmaceutique est soluble dans l'eau et est présente dans l'huile sous la forme d'une émulsion eau-dans-huile.

13. Procédé suivant la revendication 10, dans lequel le surfactant est une gomme naturelle.

14. Procédé suivant la revendication 11, dans lequel l'émulsion est formée en mélangeant l'huile dans laquelle la substance pharmaceutique est dissoute avec le surfactant, puis en ajoutant l'eau, en continuant d'agiter.

15. Procédé suivant la revendication 12, dans lequel l'émulsion est formée en mélangeant tout d'abord la substance pharmaceutique hydrosoluble avec l'huile, puis en ajoutant l'eau et le surfactant en continuant d'agiter.

16. Procédé suivant la revendication 10, dans lequel la proportion surfactant:eau:huile est d'environ 1:2:4 en volume.

17. Procédé suivant la revendication 16, dans lequel le surfactant est la gomme arabique, l'huile est une huile végétale et le polysaccharide est le dextrane présent en une quantité égale à environ quatre fois en volume la quantité d'émulsion.

18. Procédé pour préparer une composition médicinale pouvant être ingérée par voie orale suivant la revendication 1, comprenant les étapes qui consistent

a. à former une émulsion consistant essentiellement en une huile dans laquelle est dissous du palmitate de vitamine A, en gomme arabique et en eau, la proportion gomme arabique:eau:huile étant de 1:2:4 en volume,

b. à homogénéiser l'émulsion pour réduire l'huile en globules ayant un diamètre de 0,05 µm (500 Å) à 0,1 µm (1000 Å),

c. à mélanger l'émulsion homogénéisée avec une quantité de dextrane en poudre ayant un poids moléculaire moyen de 40 000 daltons équivalant à environ quatre fois le volume de l'émulsion, et

d. à encapsuler dans une capsule de gélatine une quantité du mélange émulsion-dextrane nécessaire pour apporter une dose unitaire désirée de palmitate de vitamine A.

19. Composition médicinale pouvant être ingérée par voie orale, comprenant un solvant lipidique, de l'eau et des ingrédients pharmaceutiques solubles dans les lipides, caractérisée par le fait que les ingrédients sont incorporés à une forme physique devant être ingérée par voie orale et capable de délivrer la substance pharmaceutique dans son état initial inchangé à la couche en brosse de l'intestin grêle, la forme physique des ingrédients étant comme suit:

(a) le solvant lipidique est sous la forme de globules individuels de diamètre principalement inférieur à 0,1 µm (1000 Å);

(b) chacun des globules contient en solution la substance pharmaceutique soluble dans les lipides; et

(c) chacun des globules est enveloppé dans une couche périphérique d'eau adhérant aux globules par la tension superficielle, ladite couche d'eau servant à maintenir les globules sous une forme indépendante et à ne présenter qu'une surface formée d'eau aux liquides de l'estomac.

**Revendications pour l'état Contractant: AT**

1. Procédé de préparation d'une composition médicinale pouvant être ingérée par voie orale, comprenant les étapes qui consistent:

a. à former une émulsion consistant essentiellement en une huile dans laquelle est dispersée une substance pharmaceutique, un surfactant et de l'eau,

b. à homogénéiser l'émulsion pour réduire l'huile en globules individuels contenant la substance pharmaceutique, ayant un diamètre principalement inférieur à 0,1 μm (1000 Å),

c. à mélanger l'émulsion homogénéisée avec une quantité d'un polysaccharide donnant un mélange final qui contient 80 à 90% de polysaccharide, le reste étant l'émulsion, et dans lequel les globules contenant la substance pharmaceutique sont intacts; et

d. à encapsuler le mélange en unités dosées individuelles pour obtenir une quantité dosée désirée de la substance pharmaceutique.

2. Procédé suivant la revendication 1, dans lequel la substance pharmaceutique est soluble dans les lipides et est dissoute dans l'huile.

3. Procédé suivant la revendication 1, dans lequel la substance pharmaceutique est soluble dans l'eau et est présente dans l'huile sous la forme d'une émulsion eau-dans-huile.

4. Procédé suivant la revendication 1, dans lequel le surfactant est une gomme naturelle.

5. Procédé suivant la revendication 2, dans lequel l'émulsion est formée en mélangeant l'huile contenant la substance pharmaceutique dissoute avec le surfactant, puis en ajoutant l'eau, en continuant d'agiter.

6: Procédé suivant la revendication 3, dans lequel l'émulsion est formée en mélangeant tout d'abord la substance pharmaceutique hydrosoluble avec l'huile, puis en ajoutant l'eau et le surfactant en continuant d'agiter.

7. Procédé suivant la revendication 1, dans lequel la proportion surfactant:eau:huile est d'environ 1:2:4 en volume.

8. Procédé suivant la revendication 7, dans lequel le surfactant est la gomme arabique, l'huile est une huile végétale et le polysaccharide est du dextrane présent en une quantité égale à environ quatre fois en volume la quantité d'émulsion.

9. Procédé de préparation d'une composition médicinale pouvant être ingérée par voie orale suivant la revendication 1, comprenant les étapes qui consistent

a. à former une émulsion consistant essentiellement en une huile dans laquelle est dissous du palmitate de vitamine A, en gomme arabique et en eau, la proportion gomme arabique:eau:huile étant de 1:2:4 en volume,

b. à homogénéiser l'émulsion pour réduire l'huile en globules ayant un diamètre de 0,05 μm (500 Å) à 0,1 μm (1000 Å),

c. à mélanger l'émulsion homogénéisée avec une quantité de dextrane en poudre ayant un poids moléculaire moyen de 40 000 daltons équivalent à environ quatre fois le volume de l'émulsion, et

d. à encapsuler dans une capsule de gélatine une quantité du mélange émulsion-dextrane nécessaire pour apporter une dose unitaire désirée de palmitate de vitamine A.

10. Procédé de préparation d'une composition médicinale pouvant être ingérée par voie orale, comprenant les étapes qui consistent

a. à former une émulsion consistant essentiellement en une huile dans laquelle une substance pharmaceutique est dispersée, en un surfactant et en eau, et

b. à homogénéiser l'émulsion pour réduire l'huile en globules individuels contenant la substance pharmaceutique, ayant un diamètre principalement inférieur à 0,1 μm (1000 Å), de manière que chacun des globules contenant à l'état dissous la substance pharmaceutique soluble dans les lipides soit enveloppé d'une couche périphérique d'eau adhérant au globule par la tension superficielle, ladite couche d'eau servant à maintenir les globules sous une forme indépendante et à ne présenter qu'une surface formée d'eau aux liquides de l'estomac.